# EUROPEAN PATENT APPLICATION

(11) **EP 1 394 260 A1**
(43) Date of publication of application: **03.03.2004**
(21) Application number: 03018760.3
(22) Date of filing: 27.08.2003
(51) Int. Cl.: C12P 13/04, C12P 13/16

(54) **Enzyme from Streptomyces sp. for synthesis and hydrolysis of amide, in particular amides derived from amine and fatty acid, and method for producing the enzyme**

(30) Priority: 27.08.2002 JP 2002247156
(71) Applicant: Okayama University, Okayama City, Okayama Pref. (JP)
(72) Inventor: Nakanishi, Kazuhiro, Okayama City Okayama Pref. (JP); Sakiyama, Takaharu, Okayama City Okayama Pref. (JP); Imamura, Koreyoshi, Okayama City Okayama Pref. (JP)
(74) Representative: HOFFMANN - EITLE

(57) **Abstract**

A hydrolyzing/dehydration condensing enzyme having the physical and chemical properties (1) to (3): (1) function and substrate specificity: the enzyme catalyzes a hydrolyzing or dehydration condensing reaction of an amide bond; (2) an optimal temperature range of about 55°C; and (3) an optimal pH range of 6-8.

## Description

### BACKGROUND OF THE INVENTION

### (1) Field of the Invention

This invention relates to novel enzymes, a method for producing these enzymes, and a method for synthesizing useful materials by using such an enzyme, and more particularly, the invention relates to novel enzymes for catalyzing hydrolysis and/or dehydration condensing reaction of an amide bond and a method for producing these enzymes.

### (2) Description of Related Art

There are a great number of microorganisms having an ability of producing a variety of useful proteins. As a result of significant advance and development in the studies concerning the gene engineering, the cellular engineering, the microbiology and the like, there are made reexaminations on the availability of these microorganisms.

Firstly, biocatalysts can be exemplified as a useful protein. Even if enzymes as a biocatalyst generally need a high energy such as high temperature or the like for obtaining a product by reacting a substrate, they have an excellent nature of easily and rapidly completing the reaction at about room temperature.

In general, the enzymes as the biocatalyst can be synthesized by an organic synthetic chemistry method.

However, the above organic synthetic chemistry method has a drawback that it can not be used for producing food ingredients because reagents used are not allowed for processing foods. In addition, the organic synthetic chemistry method has a drawback that it is very difficult to synthesize objective products. And also, the high energy is required in the synthesis, so that the mass production is difficult for industrial applications at a lower cost.
Therefore, it is desired to stably provide useful biocatalysts being easy in the mass production in a high yield.

### SUMMARY OF THE INVENTION

It is, therefore, an object of the invention to stably provide useful enzymes as a biocatalyst being easy in the mass production in a high yield. It is another object of the invention to provide a method for synthesizing an amide by using such an enzyme.

In order to achieve the above objects, the inventors have made investigations on various microorganisms in search of biocatalysts having excellent ability of specifically conducting hydrolysis and/or dehydration condensation, and as a result, there are discovered enzymes according to the invention and the production method thereof.

A hydrolyzing/dehydration condensing enzyme according to the invention is characterized by having the following physical and chemical properties (1) to (3):
(1) function and substrate specificity: the enzyme catalyzes a hydrolyzing and/or dehydration condensing reaction of an amide bond;
(2) an optimal temperature range of about 55°C; and
(3) an optimal pH range of 6-8.

In a preferable embodiment of the hydrolyzing/dehydration condensing enzyme according to the invention, the enzyme originates from a microorganism belonging to a Streptomyces genus.

In another preferable embodiment of the hydrolyzing/dehydration condensing enzyme according to the invention, the microorganism belonging to the Streptomyces genus is a Streptomyces mobaraensis IFO 13819 or a Streptomyces luteoreticuli IFO 13422.

A method for producing a hydrolyzing/dehydration condensing enzyme according to the invention is characterized by comprising the steps of incubating a microorganism belonging to a Streptomyces genus and having an ability of producing an enzyme for hydrolyzing/dehydration condensing an amide bond, and collecting the hydrolyzing/dehydration condensing enzyme from a culture thereof.

In a preferable embodiment of the production method according to the invention, the microorganism is a Streptomyces mobaraensis IFO 13819 or a Streptomyces luteoreticuli IFO 13422.

A method for synthesizing an amide according to the invention is characterized by reacting an amine with a fatty acid in a solvent in the presence of the aforementioned enzyme.

In a preferable embodiment of the synthesis method according to the invention, the solvent is at least one selected from the group consisting of a water soluble solvent such as glycerin, ethanol, acetonitrile or the like, a water hardly soluble organic solvent such as hexane, higher alcohol, ethyl acetate or the like and a mixed solution thereof.

A method for hydrolysis according to the invention is characterized by hydrolyzing an amide to an amine and a fatty acid in the presence of the aforementioned enzyme.

In a preferable embodiment of the hydrolysis method according to the invention, the amide is at least one selected from the group consisting of N-acyl-L-amino acid, Nε-acyl-L-Lys, N-acyl-L-peptide, Nε-L-Lys-L-peptide, antibiotic substances having an amide bond, N-(benzyloxycarbonyl)-L-amino acid, capsaicin and derivatives thereof.

### BRIEF DESCRIPTION OF THE DRAWINGS

The invention will be described with reference to the accompanying drawings, wherein:
FIG. 1 is a graph showing results of a SDS-PAGE of a purified enzyme as an embodiment of the invention;
FIG. 2 is a graph showing a pH dependency of an enzymatic activity;
FIG. 3 is a graph showing a heat stability of a purified enzyme as another embodiment of the invention;
FIG. 4 is a graph showing results in hydroxyapatite column chromatography, wherein FIGS. 4(a) and 4(b) are first and second results, respectively;
FIG. 5 is a graph showing a temperature dependency of an enzymatic activity in a purified enzyme as an embodiment of the invention;
FIG. 6 is a graph showing a history of a synthesis reaction for a capsaicin derivative; and
FIG. 7 is a graph showing a relationship between a yield and a reaction time of Nε-acyl-L-Lys.

### DETAILED DESCRIPTION OF THE INVENTION

The hydrolyzing/dehydration condensing enzyme according to the invention widely means an enzyme having an action of catalyzing the hydrolysis reaction of the amide bond and/or the dehydration condensation to the amide bond. The hydrolyzing/dehydration condensing enzyme according to the invention has the following physical and chemical properties: (1) function and substrate specificity: the enzyme catalyzes a hydrolyzing or dehydration condensing reaction of an amide bond; (2) an optimal temperature range of about 55°C; and (3) an optimal pH range of 6-8. Furthermore, the hydrolyzing/dehydration condensing enzyme according to the invention has a molecular weight of about 45 kDa to 60 kDa. This enzyme is considered to be a monomer because it exhibits a single band in an electrophoresis.

The hydrolyzing/dehydration condensing enzyme according to the invention is preferably originated from the microorganism belonging to the Streptomyces genus. More preferably, the enzyme according to the invention is originated from Streptomyces mobaraensis IFO 13819 or Streptomyces luteoreticuli IFO 13422 among the microorganisms belonging to the Streptomyces genus.

Also, the enzymatic activity of the hydrolyzing/dehydration condensing enzyme according to the invention is promoted preferably in the presence of a cobalt ion. The use of the enzyme having such a high enzymatic activity is advantageous to synthesize amides inclusive of capsaicin or capsaicin analogs in a high efficiency as mentioned later.

In the method for synthesizing an amide according to the invention is used the aforementioned hydrolyzing enzyme according to the invention. Concretely, an amine is reacted with a fatty acid in the presence of the hydrolyzing enzyme in a solvent, if necessary. For example, the reaction formula in the case of using vanillylamine as an amine is shown below.

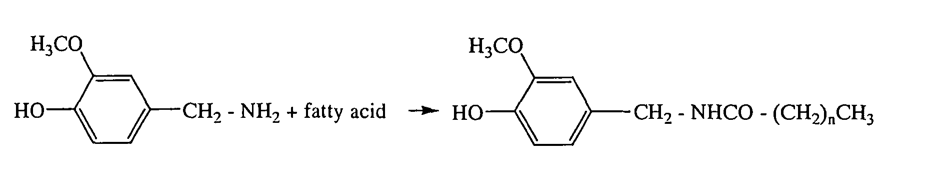

Although vanillylamine is used as an example of the amine in the above formula, various analogous amine compounds similar to vanillylamine can be used to obtain an objective capsaicin analog through the reaction with the fatty acid.

In addition to vanillylamine, various amine compounds can be used to obtain a capsaicin analog and a substance having an amide bond. For example, there may be mentioned oligopeptide, peptides including polypeptide, amino acids, antibiotic substances having an amino group and so on.

Similarly, the fatty acid is not particularly limited, and various fatty acids may be used in correspondence to the objective capsaicin analogs. For example, there may be mentioned octanoic acid, decanoic acid, lauric acid, myristic acid, palmitic acid, stearic acid and so on. Besides, various substances having a carboxyl group such as benzyloxycarboxylic acid and the like can be used.

In case of using a fatty acid having the carbon number greater than that of palmitic acid, a reaction product may not be dissolved in some solvents. Therefore, the reaction may be carried out by properly selecting a solvent. In this case, it is possible to promote the reaction by increasing the amount of the solvent or by adding a cobalt ion.

The solvent is needed or not needed in accordance with the variety of amide to be synthesized. If the reaction product is insoluble in water, the addition of the solvent is not conducted, or it is possible to control the amount of the solvent to a minimum level.

As the solvent, use may be made of a water soluble solvent such as glycerin, ethanol, acetonitrile or the like, a water hardly soluble organic solvent such as hexane, higher alcohol, acetic ether or the like, and a mixed solution thereof. Depending on a desired reaction, glycerin is preferable as the water soluble solvent, and hexane or the like is preferable as the water hardly soluble organic solvent from a viewpoint of the maintenance of enzyme stability and the high yield.

The amide applicable in the invention is not particularly limited and includes, for example, acylpeptide, antibiotic substances having an amide bond, acyl amino acid, benzyloxycarbonyl amino acid, benzyloxycarbonyl peptide, Nε-acyl lysine, Nε-acyl lysyl peptide and so on. For example, the amide may be selected from the group consisting of N-acyl-L-amino acid, Nε-acyl-L-Lys, N-acyl-L-peptide, Nε-L-Lys-L-peptide, antibiotic substances having an amide bond, N-(benzyloxycarbonyl)-L-amino acid, capsaicin and derivatives thereof.

The microorganism usable in the production of the enzyme according to the invention belongs to a Streptomyces genus capable of producing a hydrolyzing/dehydration condensing enzyme having the following physical and chemical properties:
(1) function and substrate specificity: the enzyme catalyzes hydrolyzing or dehydration condensing reaction of the amide bond;
(2) an optimal temperature range of about 55°C; and
(3) an optimal pH range of 6~8.

As mycological properties of the microorganism belonging to the Streptomyces genus, the microorganism belongs to actinomycetes and is a gram-positive obligate aerobe growing in a branched filamentous form.

From a viewpoint of the high productivity of the hydrolyzing/dehydration condensing enzyme according to the invention, the microorganism is preferable to be Streptomyces mobaraensis IFO 13819 or Streptomyces luteoreticuli IFO 13422.

Streptomyces mobaraensis IFO 13819 and Streptomyces luteoreticuli IFO 13422 are available from Institute for Fermentation, Osaka (IFO) (now transferred to "National Institute of Technology and Education Biological Resource Center" with respect to the general microorganism strain assigning business) in Japan, and IFO 13819 and IFO 13422 are the numbers of deposits.

The same strain as IF013819 has been deposited in other culture collection institutes of microorganisms. The numbers of deposits are JCM4168 in the Japan Collection of Microorganisms (JCM) of RIKEN (The Institute of Physical and Chemical Research), ATCC29032 in American Type Culture Collection (ATCC) in U. S. A., and NRRL B-3729 in National Center for Agricultural Utilization Research (NRRL) in U. S. A. The same strain as IFO 13422 has been deposited as Streptomyces mobaraensis ATCC27446 in ATCC.

The hydrolyzing/dehydration condensing enzyme according to the invention can be produced by incubating a microorganism belonging to the Streptomyces genus and having an ability of producing an enzyme for hydrolysis of amide bond in a usual manner, and collecting the enzyme from the culture thereof. Also, the hydrolyzing/dehydration condensing enzyme according to the invention can be collected from an incubated product of a spontaneous or artificial variant of the above microorganism. As a mode of the incubation, use may be made of a liquid incubation, a solid incubation and so on. In order to advantageously conduct the incubation in industry, shake incubating, ventilation stirring incubating and so on may be employed.

A nutritious source in the culture is not particularly limited, and may include a carbon source, a nitrogen source and the like usually used for incubating the microorganisms. As the carbon source, mention may be made of a yeast extract, glycerin, glucose and so on. As the nitrogen source, mention may be made of organic nitrogen compounds such as peptone, meat extract, corn steep liquor and so on. In addition, an inorganic salt such as sodium chloride, a phosphate, a sulfate or the like and a salt of a metal such as potassium, magnesium, calcium or zinc may be properly added to the culture. The incubating conditions such as incubating temperature, time and the like may be properly selected so as to adapt for the growth of the microorganism used and afford the maximum production of the hydrolyzing/dehydration condensing enzyme according to the invention. For example, pH of the culture is near neutral, preferably 6.0-8.0, more preferably around 7.0. The growing temperature of the actinomycetes is generally 28-37°C. Therefore, the incubating temperature for these microorganism bodies is preferably within a range of 28-32°C. Particularly, it is recommended in IFO that the incubating temperature for two strains: Streptomyces mobaraensis IFO 13819 and Streptomyces luteoreticuli IFO 13422 as mentioned later is 28°C.

Methods usually used for collecting metabolic products may be properly utilized to obtain the hydrolyzing/dehydration condensing enzyme according to the invention from the thus obtained culture. For example, a method utilizing a difference in solubility between the enzyme and impurities, a method utilizing an affinity, a method utilizing difference in molecular weight and the like may be used singly or in combination or repeatedly. Since the hydrolyzing/dehydration condensing enzyme according to the invention is secreted outside the microorganism, for example, the enzyme may be obtained in a purified state by the steps of incubating the microorganism, obtaining a culture supernatant through removal of the microorganism from the culture via filtration or centrifugal separation, and subjecting the supernatant to a proper combination of salting-out with ammonium sulfate, various ion exchange chromatographies, gel filtration chromatographies and so on.

The following examples are given in illustration of the invention and are not intended as limitations thereof.

### Example 1

### <Purification of hydrolyzing enzymes>

At first, there is examined whether a greater amount of a hydrolyzing enzyme is contained inside or outside microorganism bodies. When an activity is measured with respect to a solution obtained by ultrasonically crushing the microorganism bodies and a culture supernatant, the specific activity outside the microorganism bodies increases with the lapse of incubating days, whereas the activity inside the microorganism bodies is not almost observed in the incubating period of 6 days. Therefore, it is considered that the hydrolyzing enzyme according to the invention as produced by the microorganism bodies is efficiently secreted outside from the microorganism bodies.

Next, various Streptomyces species are subjected to screening so as to select those having a high hydrolyzing enzymatic activity. The screening method is as follows. In this example, capsaicin is examined as an amide.

A culture is prepared with the use of 4% beef extract, 2% polypepton and the like, and the incubation is carried out at a temperature of 30°C and an initial pH of 7.0 for 7 days under a shaking speed of 120 strokes/min. The activity is measured by HPLC after incubation with 0.13 mM capsaicin as a substrate at 37°C. Moreover, the enzymatic activity 1U is defined as an amount of the enzyme required for hydrolyzing 1 µmol of capsaicin at 37°C for 1 hour. As a result, Streptomyces mobaraensis IFO 13819 and Streptomyces luteoreticuli IFO 13422 show relatively high enzymatic activities of 1.2 U/mL and 1.0 U/mL, respectively. According to the ATCC classification, Streptomyces luteoreiticuli is classified into Streptomyces mobaraensis.

Based on the above knowledge, the incubation is conducted by using Streptomyces mobaraensis IFO 13819 as a strain. A suspension of spores of Streptomyces mobaraensis IFO 13819 is inoculated onto a liquid culture containing 2.0% of a soluble starch, 2.0% of a polypepton, 4.0% of a meat extract, 0.2% of a yeast extract and 0.2% of potassium hydrogen phosphate and 0.1% of magnesium sulfate at pH of 7 and then incubated at 30°C for 7 days.

Thereafter, ammonium sulfate is added to the culture so that a saturated concentration of an enzyme on a culture supernatant is 50% to conduct precipitation of the enzyme with ammonium sulfate. The resulting precipitates are dissolved in a buffer, fractionated with CM Sephadex C-50 and purified twice by hydroxyapatite column chromatography.

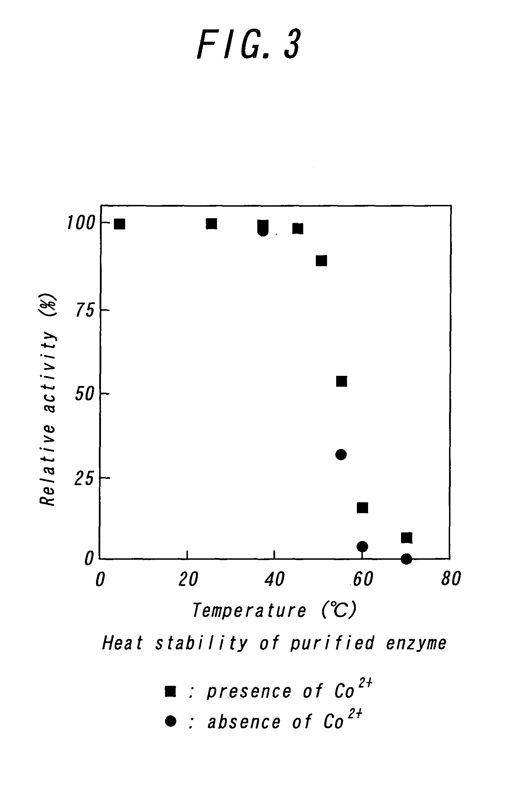

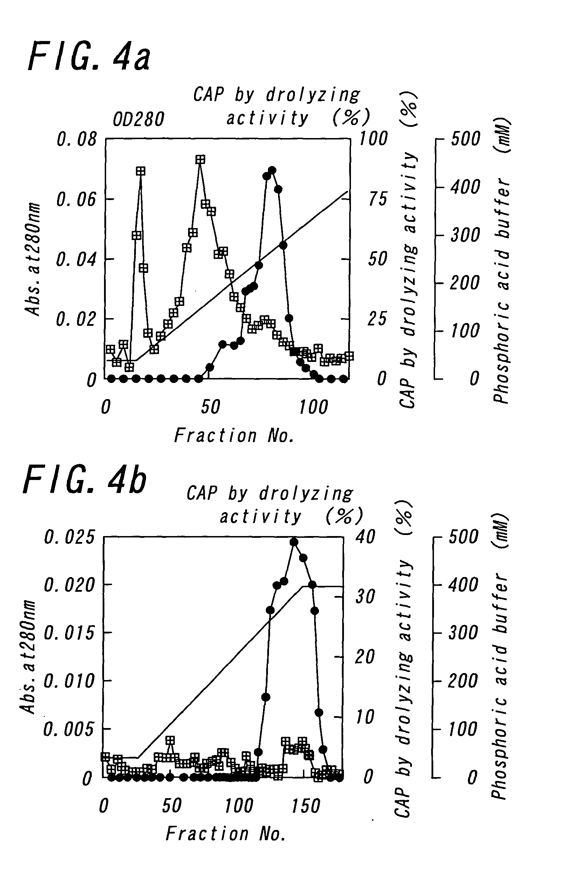

In FIG. 3 is shown an eluting curve obtained by the CM-Sephadex C-50 column chromatography. The concentration of protein and the hydrolysis activity of capsaicin (CAP-hydrolysis activity) are represented by OD280 and hydrolysis percentage, respectively. The active fraction is eluted around an NaCl concentration of 400 mM. This active fraction is further fractionated by the hydroxyapatite column chromatography to obtain an active fraction (FIG. 4(a)). Since the latter active fraction is recognized to be mixed with impurities, it is again fractioned by the hydroxyapatite column chromatography. As a result, a purified enzyme is obtained at a phosphoric acid concentration about 350 mM as shown in FIG. 4(b).

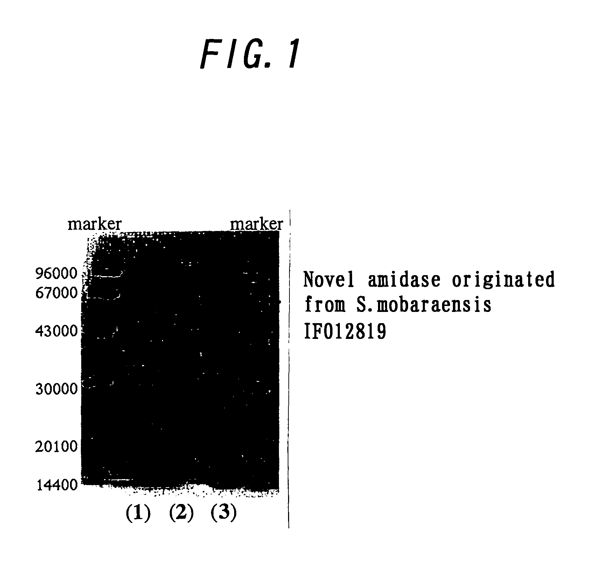

In this way, a supernatant containing the enzyme is collected by centrifugally removing the microorganism. Next, the activity of the resulting supernatant is examined. As a result, the enzyme in 0.6 L of the supernatant has an entire activity of 345 U and a specific activity of 0.061 U/mg. The enzymatic activity 1 U is defined as an amount of the enzyme required for hydrolyzing 1 µmol of capsaicin at 37°C for 1 hour. To the supernatant is added ammonium sulfate so as to be saturated at 50% , whereby a precipitated fraction is obtained. This fraction is subjected to cation exchange chromatography (CM-Sephadex) once and hydroxyapatite column chromatography twice to obtain an enzyme having a specific activity of 197.0 U/mg (Table 1), which is purified as a single enzyme (molecular weight of about 60 kDa) by electrophoresis with polyacrylamide gel (see FIG. 1). In FIG. 1, (1) represents a culture supernatant, and (2) represents 50% ammonium fraction, and (3) represents a purified enzyme. The above value of the specific activity is very high as compared with those of various enzymes having the capsaicin decomposing activity as reported by now.

**Table 1**

| | Protein (mg) | Whole activity (*units) | Yield (%) | Specific activity (U/mg) | Purified (times) |
|---|---|---|---|---|---|
| Filtrate of culture | 7475 | 345 | 100 | 0.061 | 1 |
| Ammonium sulfate | 784 | 165 | 47.8 | 0.21 | 3.4 |
| CM-SephadexC-50 | 4.8 | 62.5 | 18.1 | 13.1 | 215 |
| Hydroxyapatite (1) | 0.26 | 37.5 | 10.9 | 143.7 | 2356 |
| Hydroxyapatite (2) | 0.08 | 15.8 | 4.6 | 197.0 | 3230 |

| | | | | | |
|---|---|---|---|---|---|
| 1 Unit: amount of enzyme required for hydrolyzing 1 µmol of capsaicin at 37°C for 1 hour. | | | | | |

### <Nature of hydrolyzing enzyme>

Next, the reactivity of the purified enzyme is examined. As a result of examination on the stability against various reagents, the capsaicin hydrolysis activity of the purified enzyme is enhanced by a cobalt ion (Table 2). Also, it is shown that the activity is obstructed by adding PMSF (phenylmethane sulfonylfluoride) known as an inhibitor against serine protease.

**Table 2**

| Effects of various reagents upon enzymatic activity | | |
|---|---|---|
| | Concentration (mM) | Relative activity (%) |
| Control | | 100 |
| PCMB | 1 | 96.6 |
| Idoacetoamide | 1 | 86.5 |
| β-mercaptoethanol | 10 | 79.3 |
| DTT | 1 | 79.8 |
| MgSO₄ | 1 | 96.0 |
| FeSO₄ | 1 | 88.8 |
| CaCl₂ | 1 | 101.6 |
| AgNO₃ | 1 | 100.1 |
| ZnCl₂ | 1 | 95.5 |
| CuSO₄ | 1 | 96.9 |
| EDTA | 1 | 85.5 |
| GSH | 1 | 99.8 |
| CoCl₂ | 1 | 145.5 |
| L-Cys | 1 | 101.2 |
| (NH4) 6Mo₇O₂₄ | 1 | 88.7 |
| PMSF | 1 | 42.7 |
| Reaction solution: 0.13 mM capsaicin / 50 mM tris-HC1 (pH 7.5) | | |

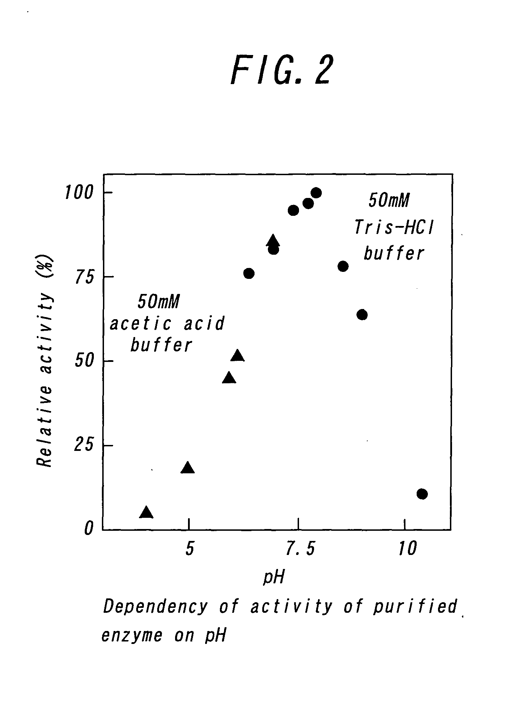

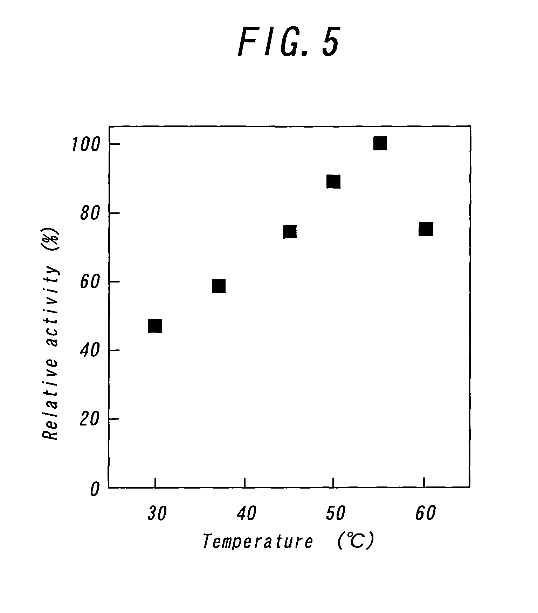

The optimal pH of the hydrolyzing enzyme is about 8 ( FIG. 2). A residual activity is examined after incubation at various temperatures for one hour with respect to a case where the cobalt ion is added and a case where no cobalt ion is added. The results are shown in FIG. 3. As to the heat resistance, the enzyme is stable up to about 50°C (FIG. 3). Above 50°C, the residual activity is higher in case of adding the cobalt ion. From these results, it is suggested that the cobalt ion can contribute to the stability of the enzyme in addition to the promotion of the activity. Further, as the temperature dependency of the activity is examined, the highest reactivity is indicated at 55°C (FIG. 5).

### Example 2

Capsaicin analogs are synthesized by using the hydrolyzing enzyme according to the invention.

An example showing an ability of the enzyme for the synthesis of the capsaicin analog is as follows. In a water/n-hexane two-phase system (hexane volume/water volume = 4) are reacted 40 mM (final concentration) of vanillylamine, and 400 mM of lauric acid in a total reaction liquid amount of 20 ml at a pH of water phase of 6.9 (a buffer solution of 100 mM tris-hydrochloric acid containing 0.5 mM of CoCl₂) for 4 days with stirring. The reaction temperature is 37°C.

As a comparative example, aminoacylase is used. The resulting capsaicin analog is quantitatively measured by HPLC analysis of the hexane phase. In Table 3 are shown reaction results when each of the enzymes is used together with various fatty acids as a substrate.

**Table 3**

| Fatty acid | Number of carbons | Synthesis of capsaicin analogs (mM) | |
|---|---|---|---|
| | | Aminoacylase | Capsaicin hydrolyzing/dehydration condensing enzyme of the invention |
| Octanoic acid | C8 | 0.74 | 0.30 |
| Decanoic acid | C10 | 0.61 | 0.43 |
| Lauric acid | C12 | 0.051 | 1.25 |
| Myristic acid | C14 | - | 0.24 |
| Palmitic acid | C16 | - | 0.01 |

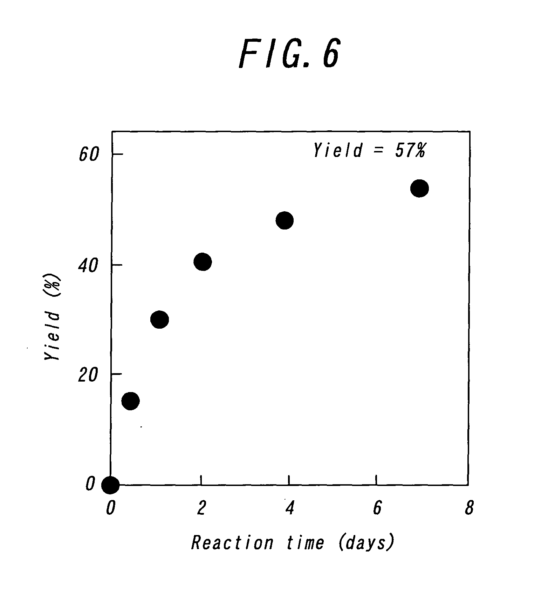

As a result, it is confirmed that the capsaicin is synthesized in a yield of about 55% with respect to the initial amount of vanillylamine (FIG. 6). Further, the enzyme according to the invention shows that the reactivity in the synthesis between vanillylamine and various saturated fatty acids as a substrate is highest in the case of lauric acid (C12), and the enzyme acts upon fatty acids having a relatively long carbon chain (palmitic acid : C16).

### Example 3

An ability of hydrolyzing and dehydration condensing by the enzyme according to the invention is examined by using N-acyl-Lamino acid as an amide. Concretely, the examination is performed by using N-acyl-L-Lys, Nα-acyl-L-Lys, and Nε-acyl-L-Lys as the N-acyl-L-amino acid. The reaction is carried out at 37°C for 30 minutes by using 0.1U of the enzyme in 0.5 ml of 50 mM tris-hydrochloric acid buffer solution containing 0.5 mM of CoCl₂ as a reaction solvent (pH: 7.5) under the condition that a final concentration of N-acyl-L-amino acid is 3.5 mM.

As a result, it is revealed that amide bond between acyl and amino with respect to all N-acyl-L-amino acids can be hydrolyzed by the enzyme according to the invention.

On the other hand, the reaction is carried out at 37°C for 24 hours by using 0.2U of the enzyme in 0.5 ml of an aqueous solution of glycerin (water content: 21%) as a solvent under the conditions of 7.5 mM of lauric acid (final concentration) and 200 mM of amino acid to attempt the synthesis of amide.

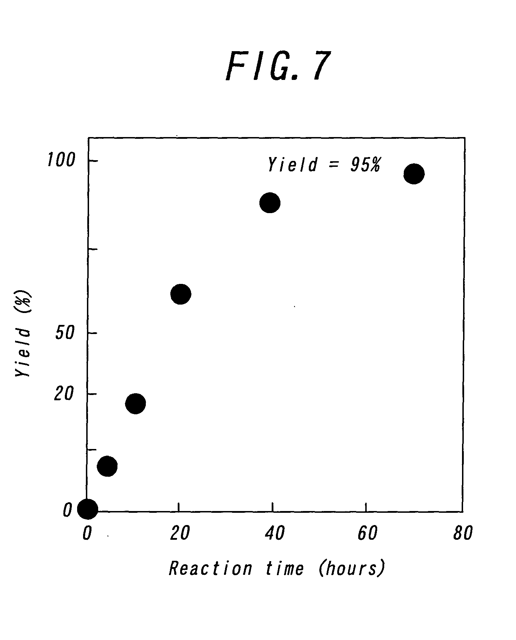

An example showing that the enzyme has an ability of synthesizing Nε-acyl-L-Lys is as follows. In 10 ml of 100 mM tris-hydrochloric acid buffer solution containing 79% of glycerin and 0.5 mM of CoCl₂ and having pH of 7.3 are reacted 7.5 mM of lauric acid and 200 mM of L-Lys by using 100 U of the enzyme for 2 days with stirring. The reaction temperature is 37°C. As a result, it is confirmed to synthesize Nε-acyl-L-Lys in a yield of about 95% with respect to the initial amount of lauric acid (FIG. 7).

Finally, it is revealed that Nα- lauryl -L-Lys and Nε-lauryl-L-Lys can be synthesized by reacting lauric acid with L-Lys.

### Example 4

An ability of hydrolyzing by the enzyme according to the invention is examined by using N-acyl-L-peptide as an amide. Concretely, the examination is performed by using N-lauryl-L-Gln-Gly, Nα-lauryl-L-Glu-L-Lys, N-lauryl-L-Lys-Gly, N-lauryl-L-Lys-L-Gln-Gly as the N-acyl-L-peptide. This is carried out in the same manner as described in Example 3 except that the final concentration of N-acyl-L-peptide as a substrate is 0.35 mM.

As a result, it is revealed that amide bond between acyl and amino with respect to all N-acyl-L-peptides can be hydrolyzed by the enzyme according to the invention. That is, lauryl acid and each of L-Gln-Gly, L-Glu-L-Lys, L-Lys-Gly, L-Lys-L-Gln-Gly are produced.

On the other hand, the synthesis of amide is attempted by carrying out by using the enzyme according to the invention under the same condition as in Example 3.

As a result, it is revealed that Nα-lauroyl-L-Lys-Gly, Nα-lauroyl-Gly-L-Lys, Nα-lauroyl-L-Lys-L-Glu, Nα-lauroyl-L-Glu-L-Lys, Nα-lauroyl-L-Met-L-Lys, Nα-lauroyl-L-Lys-L-Met, Nα-lauroyl-L-Lys-L-Val, Nα-lauroyl-L-Lys-L-Ile, Nα-lauroyl-L-Tyr-L-Lys, Nα-lauroyl-L-Lys-L-Tyr, Nα-lauroyl-L-Lys-L-Val, and Nα-lauroyl-L-His-L-Lys are synthesized by reacting lauric acid with L-Lys-Gly, Gly-L-Lys, L-Lys-L-Glu, L-Glu-L-Lys, L-Met-L-Lys, L-Lys-L-Met, L-Lys-L-Val, L-Lys-L-Ile, L-Tyr-L-Lys, L-Lys-L-Tyr, L-Lys-L-Val, and L-His-L-Lys, respectively. Also, when lauric acid is reacted with L-Lys-Gly, Gly-L-Lys, L-Lys-L-Glu, L-Glu-L-Lys and L-Tyr-L-Lys, respectively, Nε-lauroyl-L-Lys-Gly, Gly-Nε-lauroyl-L-Lys, Nε-lauroyl-L-Lys-L-Glu, L-Glu-Nε-lauroyl-L-Lys, and L-Tyr-Nε-lauroyl-L-Lys are generated, respectively, in addition to the above products.

An example showing that the enzyme has an ability of synthesizing Nε-acyl-peptide is as follows. In 10 ml of 100 mM tris-hydrochloric acid buffer solution containing 79% of glycerin and 0.5 mM of CoCl₂ and having pH of 7.0-7.5 is reacted 7.5 mM of lauric acid with each of 100 mM of L-Lys-Gly, 100 mM of Gly-L-Lys, 100 mM of L-Lys-L-Met, and 100 mM of L-Met-L-Lys by using 100 U of the enzyme for 3 days with stirring. The reaction temperature is 37°C.

As a result, Nα-lauroyl-L-Lys-Gly, Nα-lauroyl-Gly-L-Lys, Nα-lauroyl-L-Lys-L-Met, and Nα-lauroyl-L-Met-L-Lys are synthesized in yields of 30%, 20%, 33% and 42% , respectively, with respect to the initial amount of lauric acid.

### Example 5

The enzyme is examined on the hydrolysis of an antibiotic substance having an amide bond. As the antibiotic substance are used Penicillin G, Penicillin V and Ampicillin, respectively. The reaction is carried out at 37°C for 2 minutes by using 20µg of the enzyme in 0.1 ml of 50 mM tris-hydrochloric acid buffer solution containing 0.1 mM of CoCl₂ as a solvent (pH: 7.5) under a condition that a final concentration of the antibiotic substance is 10 mM.

As a result, if the hydrolyzing activity for capsaicin at pH of 7.5 is 200 U/mg (Unit is defined as an amount of the enzyme required for hydrolyzing 1µmol of the substrate at 37°C for 1 hour), the activities for Penicillin G, Penicillin V and Ampicillin are 2200 U, 6000 U and 1000 U, respectively.

In order to show the utility of the enzyme according to the invention, it is compared with the existing Penicillin Vacylase originated from Streptomyces lavendulae (Raquel Torres-Guzman et al., Biochemical and Biophysical Research Communications, Vol. 291, 593-0597 (2002)) (compared with activity kcat (1/s)). Results are shown in Table 4.

**Table 4**

| Substrate | *Streptomyces mobaraensis* (enzyme according to the invention) Activity kcat (1/s) (approximate value) | *Streptomyces lavendulae* Activity kcat (1/s) |
|---|---|---|
| Penicillin V | >80 | 60.25 |
| Penicillin G | >50 | 3.4 |
| Ampicillin | >17 | 0 |

Characteristics of Streptomyces mobaraensis (enzyme according to the invention):
It is revealed that the enzyme according to the invention is somewhat high in the activity and is wider in the substrate specificity as compared with the existing Penicilline vacylase originated from Steptomyces lavendulae. It is also revealed that the existing enzyme shows hardly an activity for Penicilline G and Ampicillin, but the enzyme according to the invention shows an excellent activity therefor.

Also, the enzyme according to the invention is compared with the conventional Urethane hydrolase IV (strain F-86) (E. Matsumura et al., Agricultural Biological Chemistry, Vol. 49, 3643-3645 (1985)). The results are shown in Table 5 (compared with Unit/mg as an activity).

**Table 5**

| Substrate | *Streptomyces mobaraensis* (enzyme according to the invention) (Unit /mg) | Urethane hydrolase IV (strain F-86) (Unit /mg) |
|---|---|---|
| Z-Gly | 0 | 108 |
| Z-Ala | 29 | 228 |
| Z-Val | 43 | 84 |
| Z-Thr | 4.5 | 162 |
| Z-Ser | 0 | 18 |
| Z-Leu | 100 | 1668 |
| Z-Ile | 28 | 18 |
| Cys(Bzl) | 250 | 228 |
| Z-Met | 85 | 294 |
| Z-Phe | 470 | 54 |
| Z-Tyr | 650 | 0 |
| Z-Trp | 230 | 0 |
| Z-Asn | 12 | 138 |
| Z-Asp | - | 360 |
| Z-Gln | 27 | 144 |
| Z-Glu | 12 | 246 |
| Z-Lys | 38 | 0 |
| Z-His | 36 | 18 |
| Z-Arg | 37 | 0 |

As a result, the enzyme according to the invention is different from the conventional Urethan hydrolase IV in the substrate specificity. It is revealed that the enzyme according to the invention shows a high activity for an aromatic amino acids such as Z-Phe, Z-Tyr and Z-Trp as opposed to the Urethane hydrolase IV having a lower activity for them.

### Example 6

An ability of hydrolyzing/dehydration condensing by the enzyme according to the invention is examined by using N-benzyloxycarbonyl-L-amino acid (Z-L-amino acid) as an amide. The reaction is performed by using 28µm of the enzyme in 0.2 ml of 50 mM tris-hydrochloric acid buffer solution containing 0.1 mM of CoCl₂ as a solvent (pH: 7.5) under a condition that a final concentration of N-benzyloxycarbonyl-L-amino acid is 10 mM at 37°C for 30 minutes (Z-L-Phe and Z-L-Tyr) or 1 hour (the other Z-L-amino acids).

As a result, it is revealed that the enzyme efficiently catalyzes the hydrolyzing reaction of N- benzyloxycarbonyl-L-amino acid (Z-L-amino acid).

Especially, the Z-L-amino acid comprising L-Phe, L-Tyr, L-Trp, L-Met, L-Leu or L-Cys(Bzl) as an amino acid is a good substrate.

The invention has been explained in detail based on the embodiments of the invention with referring to specific examples. However, the invention is not intended to be limited to the above only, but it is clear to the skilled person in the art to which the invention pertains that various modifications, changes and variations of the invention could be easily made without departing from the gist of the invention.

The invention develops an advantageous effect capable of providing enzymes which are easy in the mass production and useful for stably synthesizing amides in a higher yield as well as a method for producing amides by using such an enzyme.

## Claims

1. A hydrolyzing/dehydration condensing enzyme having the following physical and chemical properties (1) to (3):
(1) function and substrate specificity: the enzyme catalyzes a hydrolyzing or dehydration condensing reaction of an amide bond;
(2) an optimal temperature range of about 55°C; and
(3) an optimal pH range of 6-8.

2. A hydrolyzing/dehydration condensing enzyme according to claim 1, which is an enzyme originated from a microorganism belonging to a Streptomyces genus.

3. A hydrolyzing/dehydration condensing enzyme according to claim 2, wherein the microorganism belonging to the Streptomyces genus is a Streptomyces mobaraensis IFO 13819 or a Streptomyces luteoreticuli IFO 13422.

4. A method for producing a hydrolyzing/dehydration condensing enzyme, which comprises the steps of incubating a microorganism belonging to a Streptomyces genus and having an ability of producing an enzyme for hydrolyzing/dehydration condensing an amide bond, and collecting the hydrolyzing/dehydration condensing enzyme from a culture thereof.

5. The method according to claim 4, wherein the microorganism is a Streptomyces mobaraensis IFO 13819 or a Streptomyces luteoreticuli IFO 13422.

6. A method for synthesizing an amide, which comprises the step of reacting an amine with a fatty acid in a solvent in the presence of an enzyme as claimed in any one of claims 1 to 3.

7. The method according to claim 6, wherein the solvent is at least one selected from the group consisting of a water soluble solvent such as glycerin, ethanol or acetonitrile, a water hardly soluble organic solvent such as hexane, higher alcohol or acetic ether and a mixed solution thereof.

8. A method for hydrolysis, which comprises hydrolyzing an amide to an amine and a fatty acid in the presence of an enzyme as claimed in any one of claims 1 to 3.

9. The method according to claim 8, wherein the amide is at least one selected from the group consisting of N-acyl-L-amino acid, Nε-acyl-L-Lys, N-axyl-L-peptide, Nε-L-Lys-L-peptide, an antibiotic substance having an amide bond, N-(benzyloxycarbonyl)-L-amino acid, capsaicin and derivatives thereof.
